Europäisches Patentamt

European Patent Office

Office européen des brevets

(11) Veröffentlichungsnummer: **0 134 944**
**B1**

(12) # EUROPÄISCHE PATENTSCHRIFT

(45) Veröffentlichungstag der Patentschrift:
03.09.86

(21) Anmeldenummer: **84107555.9**

(22) Anmeldetag: **29.06.84**

(51) Int. Cl.⁴: **B 01 J 2/20,** B 01 J 2/26,
B 01 J 2/02, B 29 B 9/00,
A 23 G 3/02

(54) Vorrichtung zur Herstellung von Granulat.

(30) Priorität: 29.07.83 DE 3327479
05.10.83 DE 3336208

(43) Veröffentlichungstag der Anmeldung:
27.03.85 Patentblatt 85/13

(45) Bekanntmachung des Hinweises auf die Patenterteilung:
03.09.86 Patentblatt 86/36

(84) Benannte Vertragsstaaten:
BE DE FR GB IT NL

(56) Entgegenhaltungen:
DE-A-2 541 419
US-A-1 707 066
US-A-3 737 328

(73) Patentinhaber: **Santrade Ltd., Alpenquai 12 P.O.
Box 321, CH- 6002 Luzern (CH)**

(72) Erfinder: **Froeschke, Reinhard, Pestalozzistrasse 7,
D-7056 Weinstadt 2 (DE)**

(74) Vertreter: **Wilhelm, Hans- Herbert, Dr.- Ing.,
Wilhelm & Dauster Patentanwälte
Hospitalstrasse 8, D-7000 Stuttgart 1 (DE)**

EP 0 134 944 B1

## Beschreibung

Die Erfindung betrifft eine Vorrichtung zur Herstellung von Granulat aus fließfähigen, viskosen Massen, die in Tropfenform gebracht werden und erstarren, bestehend aus einem unter Druck stehenden Behälter mit mehreren Ausflußöffnungen, die intermittierend durch periodisch daran vorbeigeführte Abdeckflächen geöffnet oder geschlossen werden.

Vorrichtungen dieser Art sind bekannt (US-PS 29 79 764). Bei den bekannten Bauarten rotiert eine mit drei jeweils einen Sektor von 60° überdeckenden Flügeln versehene Scheibe am Boden eines Druckbehälters, dessen Boden perforiert ist. Durch die Drehbewegung der Flügelscheibe wird das durch den Druck aus dem Behälter durch die Perforationen zunächst streifenförmig ausfließende Material in Einzelstücke abgeschnitten, die dann die Tropfenform bilden. Nachteilig ist, daß solche Vorrichtungen nicht in Verbindung mit unter den austropfenden Massen geführten Förderbändern verwendet werden können, weil über den Querschnitt des Bandes gesehen eine ungleich große Menge von Tropfen gebildet wird, die zu einem Zusammenkleben mehrerer Tropfen führen würde. Es sind daher auch mit Walzen versehene Granuliereinrichtungen (US-PS 18 48 332 und US-PS 42 79 579) bekannt geworden, bei denen die aus mehreren Löchern austretenden Tropfen gleichmäßig auf eine Unterlage aufgegeben werden können. In dem einen Fall (US-PS 18 48 332) ist aber der Einsatz auf relativ viskose Massen beschränkt, weil dünnflüssigere Massen ungeregelt aus den Öffnungen ausfließen würden. Im zweiten Fall (US-PS 42 79 579) tritt dieser Nachteil zwar nicht auf, jedoch hat sich als nachteilig herausgestellt, daß bei hohen Produktionsgeschwindigkeiten und bei relativ niedrigen viskosen Massen der äußere rotierende und perforierte Zylinderkörper einen Teil -der Tropfenmasse mit reißt, die dann in Form von feinen Tropfen unkontrolliert auf das darunter vorbeigeführte Kühlband o.dgl. herabfallen und so zu einem unerwünschten Verkleben oder Beeinflussen der Tropfenform und/oder der Abnahme vom Band führen können.

Der Erfindung liegt daher die Aufgabe zugrunde, eine Vorrichtung zur Herstellung von Granulat der eingangs genannten Art so auszubilden, daß auch bei sehr hohen Produktions- und Tropfgeschwindigkeiten das Abspritzen von Tropfenresten vermieden wird.

Die Erfindung besteht darin, daß der Behälter ein mit einem Austrittsschlitz versehener Rohrkörper ist, der mit seiner mit dem Schlitz ausgestatteten Außenfläche an einem daran vorbeigeführten perforierten Band anliegt. Diese Ausgestaltung arbeitet zwar ebenso wie die bekannten Bauarten nach den, Prinzip des Öffnens und Verschließens von Durchtrittsöffnungen. Da der außenliegende perforierte Teil aber ein in ebener Richtung fortbewegter Körper ist, wird ein Abschleudern von Tropfenresten durch Zentrifugalkräfte vermieden. Die Länge des Bandes, die hinter dem Rohrkörper zur Verfügung steht, kann so gewählt werden, daß nahezu alle Tropfenreste bis dahin noch vom Band abgetropft sind. Wird darunter ein Förder- oder Kühlband bewegt, das parallel zu dem perforierten Band geführt ist und auch die gleiche Laufgeschwindigkeit aufweist, dann können die noch in den Perforationen befindlichen Reste bei ihrem Abtropfen nur auf darunter schon befindliche Tropfen fallen, so daß eine unerwünschte und unkontrollierte Belegung des Kühl- oder Förderbandes mit erstarrenden Massen nicht eintritt.

Eine baulich einfache Form ergibt sich, wenn das perforierte Band ein endloses Band ist, das um zwei Umlenktrommeln geführt ist, von denen eine oder beide angetrieben sind. Der Rohrkörper kann dann zweckmäßig zwischen beiden Umlenktrommeln in Abstand vor der in Bandlaufrichtung hinten liegenden Umlenktrommel am unteren Trum des Bandes angeordnet sein, so daß die Bandlänge ausreicht, um das Abtropfen in dem oben angegebenen Sinn zu gewährleisten. Durch zusätzliche Heizeinrichtungen kann dafür gesorgt werden, daß die Tropfenreste auch aus den Perforationen heraustropfen.

Die Spannung des Bandes kann in bekannter Weise einstellbar sein. Wenn dann die mit dem Band zusammenwirkende Außenfläche des Rohrkörpers etwas über die durch eine Tangente an die beiden Ümlenktrommeln bestimmte Ebene nach unten vorsteht, dann kann durch die Bandspannung ein sicheres und dichtes Anliegen des Bandes an dem Schlitz des Rohrkörpers erreicht werden. Natürlich ist es dann, wenn das perforierte Band mit gleicher Geschwindigkeit wie ein parallel darunter geführtes Kühl- oder Förderband läuft, auch möglich, mehrere Rohrkörper innerhalb des perforierten Bandes vorzusehen, weil die an einer späteren Stelle herausgedrückten Tropfen auf bereits darunter befindliche Tropfenteile fallen. Zweckmäßig wird die Außenfläche des Rohrkörpers, die mit dem Band jeweils in Berührung steht, beidseits des Schlitzes gewölbt ausgebildet. Der Rohrkörper selbst kann mit einem Führungskanal für die zu vertropfende Masse und mit mehreren in einer Reihe angeordneten Querbohrungen dazu sowie mit einer diese Querbohrungen untereinander verbindenden Nut versehen sein, die nach außen offen ist und mit ihrer in die Außenfläche mündenden Seite den Austrittsschlitz bildet. Der Rohrkörper kann auch noch mit Heiz- oder Temperierelementen versehen sein, die dafür sorgen, daß das auszutropfende Material während der Tropfenbildung aufgrund seiner Temperatur die gewünschte Viskosität aufweist. Auch seitliche Einweiser können an ihm vorgesehen werden, um seitlich zwischen Rohrkörper und Band austretendes Produkt der Perforation zuzuführen. Um schließlich eine gewisse Reinigung des perforierten Bandes während seines Umlaufes zu erreichen, können zum einen die beiden Umlenktrommeln beheizt und/oder am oberen Trum entweder Heizstrahler o.dgl. vorgesehen sein, welche das noch in den Perforationen befindliche Restmaterial zum Abtropfen zwingen. Es ist natürlich auch möglich, das gesamte Band einzuschließlich der Rohrkörper in einem entsprechend temperierten Gehäuse anzuordnen.

Bei einer solchen Vorrichtung wird die zu vertropfende Masse durch das untere Trum des endlosen Bandes herausgedrückt. Da sich dieses endlose Band und ein darunter geführtes Transport- oder Kühlband in der gleichen Richtung und vorzugsweise mit derselben Geschwindigkeit bewegen, führen Resttropfen, die im Laufe der Bandbewegung noch aus den Perforationsöffnungen nach unten fallen, nicht zu einem unkontrollierten Verspritzen des Kühl- oder Transportbandes. Das abtropfende Material fällt auf bereits vorhandene Tropfen.

Es hat sich aber gezeigt, daß es auch durch Zusatzheizeinrichtungen nicht ohne weiteres möglich ist, die Perforationsöffnungen während eines Bandumlaufes vollständig von Restmaterial zu befreien. In den Öffnungen und auch zum Teil an der Außenseite des umlaufenden perforierten Bandes hält sich ein Rest von noch flüssigen viskosen Material, der zu einer Störung des nach dem Umlauf einer Reihe von Perforationsöffnungen wieder einsetzenden Vertropfungsvorganges führen kann.

Eine Verbesserung der neuen Vorrichtung wird daher dadurch erreicht, daß auf dem oberen Trum des umlaufenden Bandes die Unterkante einer quer zur Bandbreite verlaufenden Wand aufsitzt, die an der Außenseite des Bandes noch anhaftendes Material durch die Perforierungen nach innen drückt. Auf der Außenseite vorhandenes Material wird daher zwangsweise in die Perforationen gezwungen und gelangt daher auf die Innenseite des umlaufenden Bandes, von wo aus es wieder einem erneuten Vertropfungsvorgang zugeführt werden kann. Die auf der Oberseite des Bandes aufsitzende Wand besteht zweckmäßig aus Wandflächen, die sich trichterförmig gegen die Bandlaufrichtung öffnen. Diese Wandflächen sammeln somit alles noch auf der Außenseite des Bandes anhaftende Material, führen es zur Bandmitte, wo es dann durch die Anstauwirkung durch die Perforationen nach innen gedrückt wird. Zu diesem Zweck reicht es aus, wenn die Wandflächen ebene Flächen sind, die unter einem Winkel zueinander geneigt angeordnet sind, der sich gegen die Bandlaufrichtung öffnet und der als stumpfer Winkel ausgebildet ist. Natürlich kann die Anordnung dabei so getroffen werden, daß eine symmetrische Ausgestaltung erreicht wird.

Eine weitere Verbesserung besteht darin, daß die Wandflächen so ausgebildet sind, daß im Bereich der Bandoberfläche an sie gelegte Tangenten unter einem spitzen Winkel zur Bandoberfläche geneigt sind, der sich ebenfalls gegen die Bandlaufrichtung öffnet. Durch diese Ausgestaltung wird eine Art Spachteleffekt erreicht, der dafür sorgt, daß das angestaute Material möglichst gut in die Perforationen zurückgedrückt wird. Diese Stau- und Spachtelflächen werden zweckmäßigerweise im Bereich der in der Bandlaufrichtung des oberen Trums vorderen Umlenktrommel angeordnet, damit das nach innen gedrückte Material beim Rücklauf des oberen Trumes unter der Wirkung, beispielsweise einer Zusatzheizung, genügend Zeit zum Abtropfen nach unten hat. Es kann aber auch im Bereich der in Bandlaufrichtung des oberen Trumes hinteren Umlenktrommel, eine an der Innenseite des oberen Trumes angreifende Abschabekante vorgesehen sein, die sich quer zur Bandbreite erstreckt und die mechanisch mit dazu beiträgt, daß das von den ersten Wandflächen nach innen gedrückte Material spätestens an der Stelle der Abschabekante von der Innenfläche des Bandes entfernt wird und zwangsweise auf das untere Trum gelangt. Die Abschabekante ist dabei zweckmäßig die Oberkante einer Abtropfwand, die in Laufrichtung des unteren Bandtrumes gesehen vor dem ringförmigen Körper liegt; das abgeschabte und nach unten geleitete Material gelangt so vor der Stelle, an der die Vertropfung einsetzt, wieder auf das untere Trum und wird zusammen mit dem neuen Material in Tropfenform nach unten gedrückt.

Da von den am vorderen Ende des oberen Trumes angeordneten Wandflächen, die trichterförmig angelegt sind, das meiste Material in der Bandmitte nach innen gedrückt wird, ist es vorteilhaft, wenn die Abtropfwand an ihrer Unterkante mit einer Auffangrinne für das an ihr ablaufende Material versehen ist; das in der Mitte der Abtropfwand sich ansammelnde Material kann dann über diese Rinne in der Art eines Überlaufwehres gleichmäßig auf das untere Bandtrum abgegeben werden. In der Zeichnung sind Ausführungsbeispiele der neuen Tropfenformvorrichtung gezeigt und in der nachfolgenden Beschreibung erläutert. Es zeigen:

Fig. 1 Eine schematische Seitenansicht einer neuen Vorrichtung zum Granulieren erstarrender Massen,

Fig. 2 die schematische Draufsicht auf die Vorrichtung der Fig. 1 jedoch ohne Gehäuse,

Fig. 3 eine vergrößerte und perspektivische Detaildarstellung eines zum Vertropfen vorgesehenen Rohrkörpers der Fig. 1 mit dem darunter geführten perforierten Band,

Fig. 4 einen Teilschnitt durch den Rohrkörper der Fig. 3 längs der Linie IV,

Fig. 5 die schematische Darstellung einer anderen Vorrichtung ähnlich Fig. 1,

Fig. 6 die vergrößerte Detaildarstellung des Tropfvorganges aus zwei hintereinander an einem umlaufenden Band vorgesehenen Rohrkörpern,

Fig. 7 eine schematische Darstellung des Querschnittes eines erstarrenden Tropfens, der in einer Vorrichtung gebildet wird, die gemäß Fig. 6 ausgebildet ist,

Fig. 8 eine schematische Seitenansicht einer neuen Vorrichtung zum Granulieren erstarrender oder gelierender Massen,

Fig. 9 die schematische Draufsicht auf die Vorrichtung der Fig. 8, jedoch ohne Gehäuse,

Fig. 10 eine vergrößerte Darstellung einer zweiten Ausführungsform der auch bei der Einrichtung der Fig. 8 und 9 am oberen Bandtrum vorgesehenen Stauwand und

Fig. 11 eine vergrößerte Darstellung der Einzelheit IV in Fig. 8.

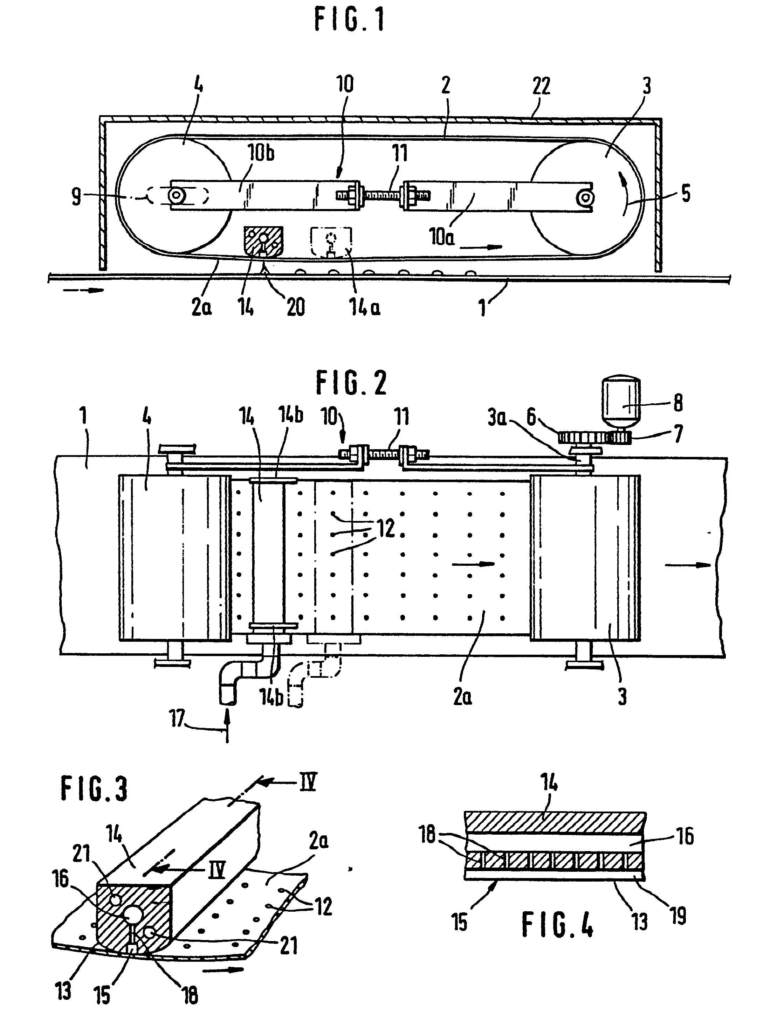

In den Fig. 1 und 2 ist oberhalb eines Förderbandes 1 ein endloses umlaufendes Band 2 an zwei Umlenktrommeln 3 und 4 geführt, die in nicht näher dargestellter Weise oberhalb des Förderbandes 1 beispielsweise in einem Traggestell gelagert sind. Die Umlenktrommel 3 ist im Sinne des Pfeiles 5 angetrieben. Das geschieht dadurch, daß ihre Lagerwelle 3a nach einer Seite verlängert ist und mit einem Zahnrad 6 versehen ist, das über ein Ritzel 7 und einen Elektromotor 8 oder über einen anderen geeigneten Antriebsmotor angetrieben ist. Die beiden Umlenktronmeln 3 und 4 sind in dem nicht gezeigten Traggestell so gelagert, daß ihr gegenseitiger Abstand in einem bestimmten Umfang einstellbar ist. Zu diesem Zweck ist die Umlenktrommel 4 in einem in der Fig. 1 gestrichelt angedeuteten Längsschlitz 9 gehalten, wobei der Abstand zwischen beiden Umlenktrommeln 3 und 9 über einen Abstandhalter 10, der gleichzeitig auch Teil des Traggestelles sein kann, eingestellt wird. Das geschieht dadurch, daß die Länge des Abstandhalters 10, der aus den Teilen 10a und 10b besteht, beispielsweise über Schraubbolzen 11 eingestellt werden kann. Solche Abstandshalter 10 sind auf beiden Seiten der Umlenktrommeln 3 und 4 vorgesehen. In Fig. 2 ist jedoch einer der beiden Abstandshalter 10 weggelassen. Durch Veränderung des Abstandes zwischen den Umlenktrommeln 3 und 4 läßt sich die Spannung des Bandes 2 einstellen, das beim Ausführungsbeispiel ein mit mehreren Öffnungen 12 perforiertes Kunststoffband, beispielsweise aus PVC, ist. Das untere Trum 2a dieses PVC-Bandes, das aus Fig. 2 zu erkennen ist, wo das obere Trum weggelassen ist, liegt mit seiner Innenseite, siehe auch Fig. 3, an der gewölbten Außenfläche 13 eines Rohrkörpers 14 an, der auf der dem unteren Trum 2a zugewandten Außenfläche 13 mit einem quer zum Band 2 verlaufenden Schlitz 15 versehen ist, der von dem dicht anliegenden perforierten Band 2 abgeschlossen oder, wenn jeweils eine Reihe von Öffnungen 12 vorbeigeführt wird, durch diese Öffnungen freigegeben wird. Der Rohrkörper 14 ist ebenso wie die Umlenktrommeln 3 und 4 fest über dem Förderband 1, beispielsweise in einem Traggestell gehalten, das in den Fig. 1 und 2 aber nicht gezeigt ist.

Der Rohrkörper 14 besitzt einen Führungskanal 16 für eine viskose und fließfähige Masse, die in Richtung des Pfeiles 17 in fließfähigem Zustand unter Druck zugeführt wird. Von diesem Führungskanal 16 aus gehen Querbohrungen 18 ab, die in eine Nut 19 münden, die zur Außenfläche 13 hin offen ist und mit ihrer Mündung den Schlitz 15 bildet. Wird der Führungskanal 16 mit dem zu vertropfenden Material unter Druck beschickt, so tritt das Material über die Querbohrungen 18 in die Nut 19 über, und zwar, wegen der Anordnung der als Drosselbohrungen wirkenden Querbohrungen 18 gleichmäßig über die Breite der Nut 19 verteilt. Von hier aus tropft das Material dann in einzelnen Tropfen 20 nach unten auf das Förderband 1, das beispielsweise als Kühlband ausgebildet sein kann und das in heißem Zustand tropfenförmig auf seine Oberfläche gelangende Material anschließend zu kleinen linsenförmigen Körpern erstarren läßt.

Um innerhalb der Rohrkörper 14 die Temperatur der zu vertropfenden Masse aufrechtzuerhalten, können parallel zu dem Führungskanal 16 entweder Strömungskanäle 21 vorgesehen sein, durch die ein temperiertes Thermoöl fließt. Es ist natürlich auch möglich, diese Strömungskanäle durch beispielsweise Elektroheizelemente zu ersetzen. Um zu vermeiden, daß Produktmasse, die seitlich zwischen Rohrkörper 14 und dem Band 2 austritt, seitlich vom Band 2 herunterläuft, ist der Rohrkörper 14 mit Einweisern 14b in der Form von radial abstehenden Rippen versehen, die das produkt auf die Bandinnenfläche und auf die Perforationsöffnungen 12 zurückdrängen.

Das endlose Band 2 ist, wie aus Fig. 1 hervorgeht, von einem Gehäuse 22 umgeben, in dessen Innenraum eine Temperatur aufrechterhalten werden kann, welche die Fließfähigkeit und damit die Tropffähigkeit des zu verarbeitenden Materials gewährleistet.

Aus Fig. 1 ist auch erkennbar, daß der Rohrkörper 14, der, wie später noch erläutert werden wird, auch durch weitere Rohrkörper, z.B. 14a, ergänzt werden kann, im Bereich der Umlenktrommel 4 angeordnet ist, so daß die Perforationen 12 des unteren Trums 2a des Bandes 2 noch eine große Strecke parallel zum Förderband 1 zu durchlaufen haben, ehe sie der Umlenktrommel 3 zugeführt werden.

Restmaterial, das daher noch in den Öffnungen 12 hängt und nicht nach unten abgetropft ist, hat daher auf dem ganzen Weg von dem Rohrkörper 14 bis zur Umlenktrommel 3 noch Gelegenheit nach unten abzutropfen. Um zu gewährleisten, daß diese Resttropfen nicht unkontrolliert auf das Förderband 1 fallen, ist die Geschwindigkeit des Bandes 2 gleich groß gewählt. Das bedeutet, daß Resttropfen aus den Perforationsöffnungen 12 auf dem ganzen Weg des unteren Trums 2a vom Rohrkörper 14 bis zur Umlenktrommel 3 jeweils auf bereits darunter befindliche größere Tropfen fallen und dort mit diesem Material zusammen zu einem etwas größeren Tropfen verlaufen. Unerwünschte Spritzer auf dem Förderband 1 treten daher nicht auf. Bei der Umlenkung an der Umlenktrommel 3 haben sich die Öffnungen 12 weitgehend selbst gereinigt, so daß dort aufgrund der Zentrifugalkräfte kein Abschleudern von Material mehr auftritt. diese Ausgestaltung ermöglicht es daher, das Band 2 mit verhältnismäßig großer Geschwindigkeit laufen zu lassen, so daß die neue Vorrichtung eine sehr große Produktionskapazität bei niedrig viskosen Massen hat.

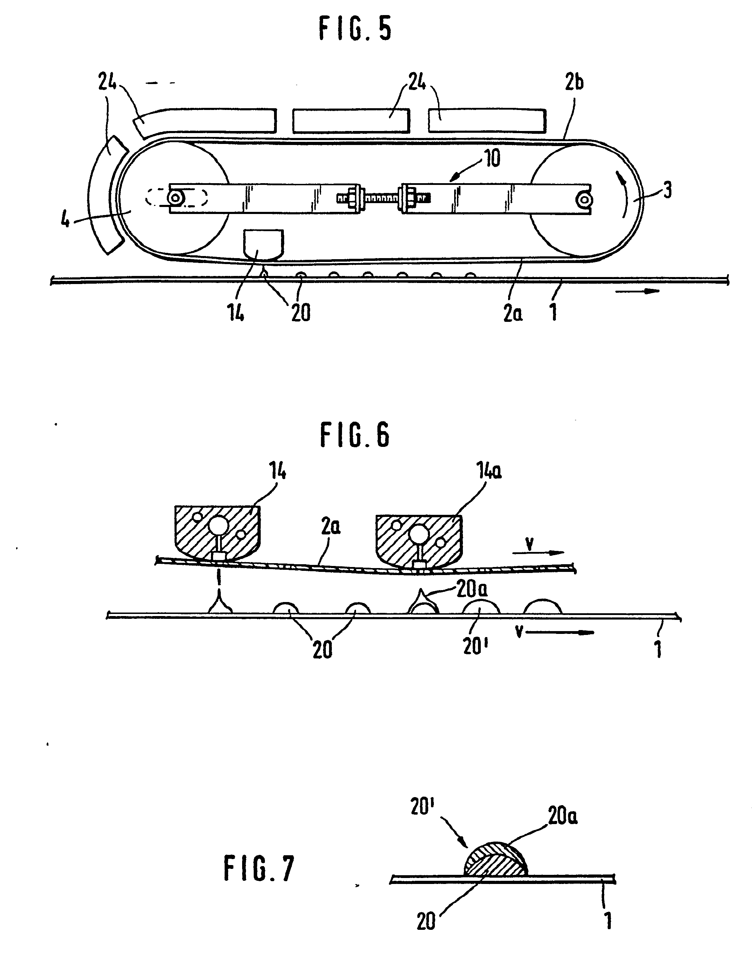

Die Fig. 5 bis 7 zeigen im Prinzip die gleiche Ausbildung wie Fig. 1. Dort sind allerdings Infrarotstrahler 24 o. dgl. im Bereich des oberen Trumes 2b des Bandes 2 vorgesehen, die dafür sorgen, daß an dieser Stelle durch intensive Aufheizung allenfalls noch vorhandenes Restmaterial aus den Öffnungen 12 entfernt wird, ehe diese wieder dem Rohrkörper 14 zugeführt werden.

Wird gemäß Fig. 6 vorgesehen, einem Rohrkörper 14 noch einen zweiten nachzuschalten, so kann der Durchsatz an zu vertropfendem Material erhöht werden. Auch in diesem Fall fallen die aus den zweiten Rohrkörper 14a austretenden Tropfen 20a auf die bereits vom ersten Rohrkörper 14 ausgetropften Tropfen 20 und verbinden sich mit diesen zu größeren Tropfen 20°, die, je nach Viskosität, die Form von mehr oder weniger hohen Kugelabschnitten erhalten und in dieser Form erstarren, wenn sie von dem Förderband 1 weiter befördert werden, das beispielsweise in einen Kühltunnel laufen kann oder unmittelbar als Kühlband ausgebildet sein kann, das beispielsweise aus Stahl besteht und von unten her mit einer Kühlsole besprüht wird. Diese Kühlung setzt hinter dem Bereich der Tropfenformeinrichtung ein. Ebenso wie die Tropfen 20a eines zweiten Rohrkörpers 14a vereinigen sich auch Resttropfen aus den Perforationsöffnungen 12 mit den Tropfen 20. Die Fortbewegungsgeschwindigkeit des Bandes 2 und des parallel dazu angeordneten Förderbandes 1 müssen zu diesem Zweck gleich groß gewählt sein.

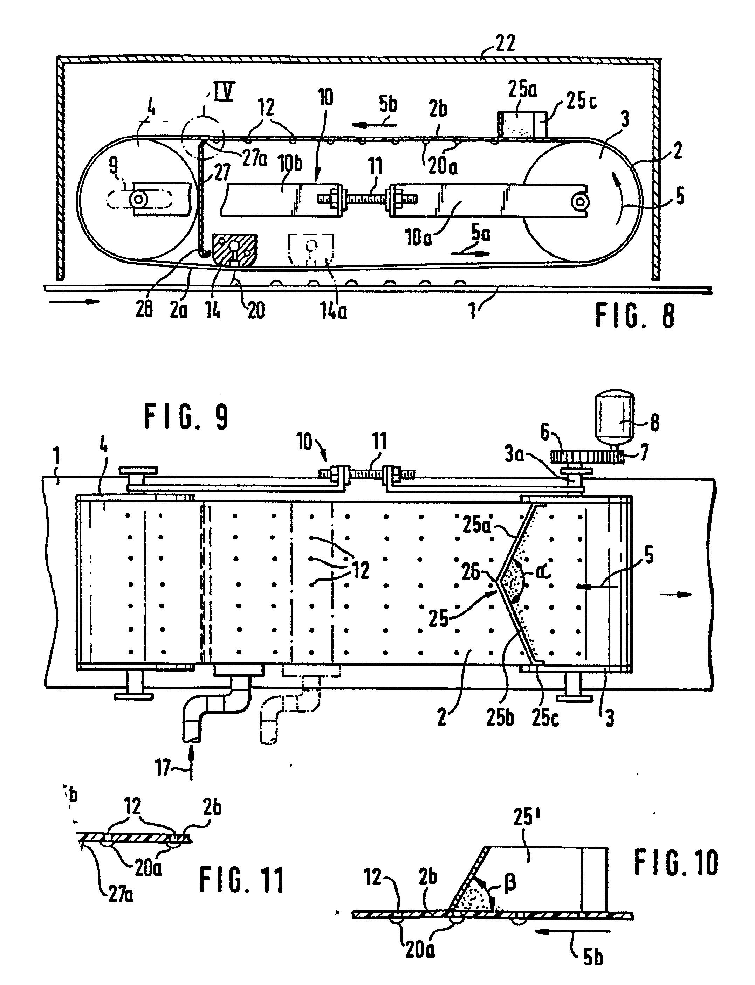

In den Fig. 8 und 9 ist - wie auch in den vorhergehenden Figuren oberhalb eines Förder- oder Kühlbandes 1 ein endlos umlaufendes Band 2 an zwei Umlenktrommeln 3 und 4 geführt, die in nicht näher dargestellter Weise oberhalb des Förderbandes 1 beispielsweise in einem Traggestell gelagert sind. Die Umlenktrommel 3 ist im Sinne des Pfeiles 5 angetrieben; das geschieht dadurch, daß ihre Lagerwelle 3a nach einer Seite verlängert ist und mit einem Zahnrad 6 versehen ist, das über ein Ritzel 7 und einen Elektromotor 8 oder über einen anderen geeigneten Antriebsmotor angetrieben ist. Die beiden Umlenktrommeln 3 und 4 sind in dem nicht gezeigten Traggestell so gelagert, daß ihr gegenseitiger Abstand in einem bestimmten Umfang einstellbar ist. Zu diesem Zweck ist die Umlenktrommel 4 in einem in der Fig. 8 gestrichelt angedeuteten Längsschlitz 9 gehalten, wobei der Abstand zwischen beiden Umlenktrommel 3 und 4 über einen Abstandshalter 10, der gleichzeitig auch Teil des Traggestelles sein kann, eingestellt wird. Das geschieht dadurch, daß die Länge des Abstandhalters 10, der aus den Teilen 10a und 10b besteht, beispielsweise über Schraubbolzen 11 eingestellt wird. Solche Abstandshalter 10 sind auf beiden Seiten der Lenktrommeln 3 und 4 vorgesehen. In Fig. 9 ist jedoch einer der beiden Abstandshalter 10 weggelassen. Durch Veränderung des Abstandes zwischen den Umlenktrommeln 3 und 4 läßt sich die Spannung des Bandes 2 einstellen, das beim Ausführungsbeispiel ein mit mehreren Öffnungen 12 perforiertes Kunststoffband, beispielsweise aus PVC ist. Das untere Trum 2a dieses PVC-Bandes liegt mit seiner Innenseite an der gewölbten Außenfläche eines Rohrkörpers 14 an, der auf der dem unteren Trum 2a zugewandten Außenfläche mit einem quer zum Band 2 verlaufenden Schlitz versehen ist, der von dem dicht anliegenden perforierten Band 2 abgeschlossen oder wenn jeweils eine Reihe von öffnungen 12 vorbeigeführt wird, durch diese Öffnungen freigegeben wird.

Der Rohrkörper 14 ist ebenso wie die Umlenktrommeln 3 und 4 fest über dem Förderband 1, beispielsweise in dem Traggestell, gehalten, das in den Fig. 8 und 9 aber nicht gezeigt ist.

Wird der Rohrkörper 14, wie bei den Ausführungsformen der vorhergehenden Figuren, mit dem zu vertropfenden Material unter Druck beschickt, so tritt das Material in Form von Tropfen 20 durch die öffnungen 12 des Bandes 2 nach unten aus und fällt in Tropfen 20 auf das Förderband 1. Das endlose Band 2 ist von einem Gehäuse 22 umgeben, in dessen Innenraum eine Temperatur aufrechterhalten werden kann, welche die Fließfähigkeit und damit die Tropffähigkeit des zu verarbeitenden Materials gewährleistet. Der Rohrkörper 14 kann auch durch weitere Rohrkörper 14a ergänzt werden, wenn die Produktion erhöht werden soll.

Um zu erreichen, daß beim Abtropfvorgang an der Außenseite des umlaufenden Bandes 2 noch anhaftendes Material entfernt wird, ehe es den neuen Vorgang der Tropfenbildung störend beeinflussen kann, ist auf der Oberseite des oberen Trumes 2b des Bandes 2 eine quer zum Band 2 verlaufende Stauwand 25 vorgesehen, die aus zwei Wandflächen 25a und 25b besteht,die unter einem stumpfen Winkel α, der sich gegen die Bandlaufrichtung 5 öffnet, zueinander geneigt angeordnet sind und sich daher trichterförmig gegen die Bandlaufrichtung öffnet. An den beiden Außenkanten des Bandes 2 sind jeweils in Bandlaufrichtung 5 verlaufende Endwandstücke 25c vorgesehen. Der Scheitel 26 des Winkels α liegt in der Mitte des Bandes. Die Anordnung der Wandflächen 25a und 25b ist daher symmetrisch zur Mitte des Bandes 2. Die Unterkanten der Wandflächen 25a und 25b liegen fest an der Oberseite des oberen Trumes 2b des Bandes an, so daß an der Außenseite des Bandes 2 noch anhaftendes Material sich innerhalb der trichterförmigen Stauwand 25 sammelt, und zwar vorwiegend in der Bandmitte. Dieses Material wird durch diesen Anstauvorgang durch die Öffnungen 12 im oberen Trum 2b heireingedrückt, fällt dann entweder auf das untere Trum 2a herab oder verbleibt in Form von Tropfen 20a an der Unterseite des oberen Trumes 2b. Um diese Tropfen 20a oder anderes, an der Innenseite anhaftendes Material vor dem Erreichen der Umlenktrommel 4 sicher zu entfernen, ist im Inneren des umlaufenden Bandes 2 im Bereich der Umlenktrommel 4 eine Abtropfwand 27 vorgesehen, die sich ebenfalls quer zur Breite des Bandes 2 erstreckt und die eine Abschabekante 27a besitzt, die, wie insbesondere aus Fig. 11 hervorgeht, gegen die Laufrichtung 5b am oberen Trum 2b gerichtet sein kann und die dafür sorgt, daß die an der Unterseite des oberen Trumes 2b noch anhaftenden Tropfen 20a oder anderes Material an der Abtropfwand 27 nach unten geführt wird, und zwar noch vor dem Erreichen der Umlenktrommel 4. Das nach unten laufende Material gelangt dann in eine an der Unterkante der Abtropfwand 27 vorgesehene Auffangrinne 28, die dafür sorgt, daß sich das - wegen der trichterförmigen Anordnung der Stauwand 25 - am meisten in der Mitte der Abtropfwand 27 ansammelnde Material wieder gleichmäßig über die Breite des Bandes 2 verteilt und von der Auffangrinne 28 aus, wie bei einem Überfallwehr, auf das untere Trum 2a gegeben wird, und zwar vor den ersten Rohrkörper 14, der dann wiederum dafür sorgt, daß auch dieses Restmaterial zusammen mit neuem Material zu Tropfen 20 geformt wird, die dann auf das Band 1 fallen.

Fig. 10 zeigt noch eine Abwandlung der Stauwand 25 der Fig.8 und 9. Die Stauwand 25' der Fig. 10 ist unter einem spitzen Winkel ß gegenüber der Oberfläche des oberen Trumes 2b des Bandes 2 geneigt. Die Stauwand 25' besteht dabei ebenfalls aus ebenen Bandflächen. Diese schräge Anordnung verbessert in der Art eines Spachtels den Eindrückeffekt der Stauwand auf das sich an dieser Wand ansammelnde Material, das, ebenso wie in Fig. 8 und 9, punktiert angedeutet ist. Natürlich wäre es auch möglich, die Stauwand 25' als eine konvex gegen die Laufrichtung 5b des oberen Trumes gewölbte Wand auszubilden, die dann eine unter einem spitzen Winkel zur Oberfläche des oberen Trumes 2b verlaufende Tangente aufweisen würde. Die gezeigte Ausführung ist aber wesentlich einfacher und ebenfalls wirkungsvoll. Die Stauwand müßte auch nicht aus zwei in einem Scheitelpunkt 26 zusammenlaufenden ebenen Wandteilen bestehen; natürlich kann die Stauwand auch gewölbt sein. Auch dann soll sie sich trichterförmig gegen die Laufrichtung 5b am oberen Trum öffnen, damit sich das anstauende Material sammelt und nicht seitlich vom Band heruntergedrückt wird.

## Patentansprüche

1. Vorrichtung zur Herstellung von Granulat aus fließfähigen, viskosen Massen, die in Tropfenform gebracht werden und erstarren oder gelieren, bestehend aus einem unter Druck stehenden Behälter mit mehreren Ausflußöffnungen, die intermittierend durch periodisch daran vorbeigeführte Abdeckflächen geöffnet oder geschlossen werden, dadurch gekennzeichnet, daß der Behälter ein mit einem Austrittsschlitz (15) versehener Rohrkörper (14) ist, der mit seiner mit dem Schlitz (15) ausgestatteten Außenfläche (13) an einem daran vorbeigeführten perforierten Band (2) anliegt.

2. Vorrichtung nach Anspruch 1, dadurch gekennzeichnet, daß das perforierte Band (2) ein endloses Band ist, das um zwei Umlenktrommeln (3 und 4) geführt ist, von denen eine angetrieben ist.

3. Vorrichtung nach den Ansprüchen 1 und 2, dadurch gekennzeichnet, daß der Rohrkörper (14) zwischen beiden Umlenktrommeln (3 und 4) in Abstand vor der in Bandlaufrichtung hinteren Umlenktrommel (3) am unteren Trum (2a) des Bandes (2) angeordnet ist.

4. Vorrichtung nach den Ansprüchen 1 bis 3, dadurch gekennzeichnet, daß die Spannung des Bandes (2) einstellbar ist und daß die mit dem Band zusammenwirkende Außenfläche (13) des Rohrkörpers (14) etwas über die durch eine Tangente an die beiden Umlenktrommeln (3, 4) bestimmte Ebene nach unten vorsteht.

5. Vorrichtung nach einem der Ansprüche 1 bis 4, dadurch gekennzeichnet, daß unterhalb des unteren Bandtrumes (2a) ein Förderband (1) für die abtropfenden Massen vorgesehen ist, das mit der gleichen Geschwindigkeit (v) wie das perforierte Band (2) und parallel zu dessen unteren Trum (2a) läuft.

6. Vorrichtung nach Anspruch 5, dadurch gekennzeichnet, daß mehrere Rohrkörper (14, 14a) innerhalb des perforierten Bandes (2) vorgesehen sind.

7. Vorrichtung nach einem, der Ansprüche 1 bis 6, dadurch gekennzeichnet, daß das perforierte Band (2) ein Kunststoffband, vorzugsweise ein armiertes PVC-Band, ist.

8. Vorrichtung nach Anspruch 1, dadurch gekennzeichnet, daß die Außenfläche (13) des Rohrkörpers (14) die mit dem Band (2) in Berührung steht, beidseits des Schlitzes (15) gewölbt ausgebildet ist.

9. Vorrichtung nach Anspruch 1, dadurch gekennzeichnet, daß der Rohrkörper (14) mit einem Führungskanal (16) für die zu vertropfende Masse, mit mehreren in einer Reihe angeordneten Querbohrungen (18) dazu und mit einer diese Querbohrungen untereinander verbindenden Nut (19) versehen ist, die nach aussen offen ist und mit ihrer in die Außenfläche (13) mündenden Seite den Austrittsschlitz (15) bildet.

10. Vorrichtung nach Anspruch 9, dadurch gekennzeichnet, daß parallel zu dem Führungskanal (16) Strömungskanäle (21) für Thermoöl oder andere Heizelemente zur Temperierung der austretenden Masse angeordnet sind.

11. Vorrichtung nach einem der Ansprüche 1 bis 10, dadurch gekennzeichnet, daß der Rohrkörper (14) mit Einweisern (25) versehen ist, welche seitlich zwischen Rohrkörper (14) und Band (2) austretendes Produkt der Perforation zuführen.

12. Vorrichtung nach einem oder mehreren der Ansprüche 1 bis 11, dadurch gekennzeichnet, daß das perforierte Band (2) und der oder die Rohrkörper (14, 14a) von einem Gehäuse (22) umgeben sind, dessen Innenraum temperaturgesteuert ist.

13. Vorrichtung nach einem der Ansprüche 1 bis 12, dadurch gekennzeichnet, daß dem oberen Trum (2b) des perforierten Bandes (2) und der in Laufrichtung am Ende dieses Trums liegenden Umlenktrommel (4) Heizstrahler (24) zugeordnet sind.

14. Vorrichtung nach einem der Ansprüche 1 bis 13, dadurch gekennzeichnet, daß beide Umlenktrommeln (3 und 4) von einem Heizmedium durchströmt werden.

15. Vorrichtung nach den Ansprüchen 1 und 2, dadurch gekennzeichnet, daß auf dem oberen Trum (2b) des Bandes (2) die Unterkante einer quer zur Bandbreite verlaufenden Wand (25, 25') aufsitzt, die an der Außenseite des Bandes (2) noch anhaftendes Material durch die Perforierungen (12) nach innen drückt.

16. Vorrichtung nach Anspruch 15, dadurch gekennzeichnet, daß die Wand (25) aus Wandflächen (25a, 25b) besteht, die sich trichterförmig gegen die Laufrichtung (5b) des oberen Bandtrumes (2b) öffnet.

17. Vorrichtung nach Anspruch 16, dadurch gekennzeichnet, daß die Wandflächen (25a, 25b) ebene Flächen sind, die unter einem Winkel (α) zueinander geneigt angeordnet sind.

18. Vorrichtung nach Anspruch 17, dadurch gekennzeichnet, daß der Winkel (α) ein stumpfer Winkel ist.

19. Vorrichtung nach einem der Ansprüche 16 bis 18, dadurch gekennzeichnet, daß der Scheitel (26) des Winkels (α) in der Mitte des Bandes liegt und daß beide Wandflächen (25a, 25b) symmetrisch zur Bandmitte verlaufen.

20. Vorrichtung nach den Ansprüchen 15 und 16, dadurch gekennzeichnet, daß die Wandflächen (25') so ausgebildet sind, daß im Bereich der Wandoberfläche an sie gelegte Tangenten unter einem spitzen Winkel (β) zur Wandoberfläche geneigt sind, der sich gegen die Laufrichtung (5b) des oberen Bandtrumes (2b) öffnet.

21. Vorrichtung nach Anspruch 20, dadurch gekennzeichnet, daß die Wandflächen (25') eben sind und als solche geneigt zur Bandoberfläche Stehen.

22. Vorrichtung nach einem der Ansprüche 15 bis 21, dadurch gekennzeichnet, daß die Wandflächen (25a, 25b, 25') im Bereich der in der Laufrichtung (5b) des oberen Trumes (2b) vorderen Umlenktrommel (3) angeordnet sind.

23. Vorrichtung nach einem der Ansprüche 15 bis 21, dadurch gekennzeichnet, daß im Bereich der in der Laufrichtung (5b) des oberen Trumes (2b) hinteren Umlenktrommel (4) eine an der Innenseite des oberen Trumes angreifende Abschabekante (27a) vorgesehen ist, die sich quer zur Laufrichtung (5b) erstreckt.

24. Vorrichtung nach Anspruch 23, dadurch gekennzeichnet, daß die Abschabekante (27a) die Oberkante einer Abtropfwand (27) ist, die - in Laufrichtung (5a) des unteren Bandtrumes (2a) gesehen - vor dem rohrförmigen Körper (14) liegt.

25. Vorrichtung nach den Ansprüchen 23 und 24, dadurch gekennzeichnet, daß die Abtropfwand (27) an ihrer Unterkante mit einer Auffangrinne (28) für das an ihr ablaufende Material versehen ist.

**Claims**

1. Apparatus for producing granular material from flowable viscous compositions which are brought to drop form and set or jellify, consisting of a pressured container having a plurality of outflow orifices which are intermittently opened or closed by masking surfaces which periodically pass over them, characterized in that the container is a tubular body (14) provided with an outlet slot (15), its outer face (13) in which the slot (15) is provided bearing on a perforated belt (2) which passes by it.

2. Apparatus according to Claim 1, characterized in that the perforated belt (2) is an endless belt which is passed over two reversing drums (3 and 4), one of which is driven.

3. Apparatus according to Claims 1 and 2, characterized in that the tubular body (14) is disposed between both reversing drums (3 and 4) at a distance from and before the (in the direction of belt run) rear reversing drum (3) on the bottom strand (2a) of the belt (2).

4. Apparatus according to Claims 1 to 3, characterized in that the tension of the belt (2) is adjustable and in that the outer surface (13) of the tubular body (14) which cooperates with the belt projects somewhat downwardly beyond the plane defined by a tangent on the two reversing drums (3, 4).

5. Apparatus according to one of Claims 1 to 4, characterized in that for the compositions which drip off, there is beneath the lower belt strand (2a) a conveyor belt (1) which runs at the same speed (v) as and parallel with the bottom strand (2a) of the perforated belt (2).

6. Apparatus according to Claim 5, characterized in that a plurality of tubular bodies (14, 14a) are provided within the perforated belt (2).

7. Apparatus according to one of Claims 1 to 6, characterized in that the perforated belt (2) is a synthetic plastics belt, preferably a reinforced PVC belt.

8. Apparatus according to Claim 1, characterized in that the outer surface (13) of the tubular body (14) which is in contact with the belt (2) is constructed to be bulbous on both sides of the slot (15).

9. Apparatus according to Claim 1, characterized in that the tubular body (14) is provided with a guide passage (16) for the composition which is to be converted to drop form, the said guide passage having disposed in a row a plurality of transverse bores (18) and a groove (19) connecting these transverse bores to one another, the groove being outwardly open, its side which discharges into the outer surface (13) constituting the discharge slot (15).

10. Apparatus according to Claim 9, characterized in that there are parallel with the guide passage (16) flow passages (21) for heating oil or other heating elements for adjusting the temperature of the emerging composition.

11. Apparatus according to one of Claims 1 to 10, characterized in that the tubular body (14) is provided with guides (25) which feed to the perforation stage product emerging laterally between the tubular body (14) and the belt (2).

12. Apparatus according to one or more of Claims 1 to 11, characterized in that the perforated belt (2) and the tubular body or bodies (14, 14a) are enclosed by a housing (22), the interior of which is temperature controlled.

13. Apparatus according to one of Claims 1 to 12, characterized in that radiant heaters (24) are associated with the upper strand (2b) of the perforated belt (2) and the reversing drum (4) which, in the direction of movement, is disposed at the end of this strand.

14. Apparatus according to one of Claims 1 to 13, characterized in that a heating medium flows through both reversing drums (3 and 4).

15. Apparatus according to Claims 1 and 2, characterized in that seated on the upper strand (2b) of the belt (2) is the bottom edge of a wall (25, 25') extending transversely of the width of the belt and which forces inwards through the perforations (12) any material which is still clinging to the outside of the belt (2).

16. Apparatus according to Claim I5, characterized in that the wall (25) consists of wall surfaces (25a, 25b) which open out in a funnel shape against the direction of movement (5b) of the upper belt strand (2b).

17. Apparatus according to Claim 16, characterized in that the wall faces (25a, 25b) are flat faces which are disposed at an angle (α) to each other.

18. Apparatus according to Claim 17, characterized in that the angle (α) is an obtuse angle.

19. Apparatus according to one of Claims 16 to 18, characterized in that the apex (26) of the angle (α) lies in the middle of the belt and in that both wall faces (25a, 25b) extend symmetrically to the centre of the belt.

20. Apparatus according to Claims 15 and 16, characterized in that the wall faces (25') are so constructed that tangents laid on them in the region of the wall surface are inclined at an acute angle (β) to the wall surface, the angle opening out towards the direction of movement (5b) of the upper belt strand (2b).

21. Apparatus according to Claim 20, characterized in that the wall faces (25') are flat and as such are inclined in relation to the belt surface.

22. Apparatus according to one of Claims 15 to 21, characterized in that the wall faces (25a, 25b, 25') are located in the region of the reversing drum (3) which is in front in the direction of movement (5b) of the upper strand (2b).

23. Apparatus according to one of Claims 15 to 21, characterized in that there is in the region of the reversing drum (4) which is at the rear in the direction of movement (5b) of the upper strand (2b) a scraper edge (27a) which engages the inside face of the upper strand and which extends transversely of the direction of movement (5b).

24. Apparatus according to Claim 23, characterized in that the scraper edge (27a) is the top edge of a dropper wall (27) which, viewed in the direction of movement (5a) of the lower belt strand (2a), is located in front of the tubular body (14).

25. Apparatus according to Claims 23 and 24, characterized in that the dropper wall (27) is provided on its bottom edge with a gutter (28) for material running of it.

## Revendications

Appareil pour produire du granulé à partir de masses visqueuses capables de s'écouler, qui sont mises en forme de gouttes et se solidifient ou se gélifient, comprenant un récipient sous pression avec des orifices d'écoulement qui sont ouverts et fermés de façon intermittente par des surfaces de recouvrement déplacées périodiquement devant les orifices, caractérisé en ce que le récipient est une pièce tubulaire (14) pourvue d'une fente de sortie (15), qui s'applique par sa surface externe (13), présentant la fente (15), contre une bande perforée (2) guidée devant cette surface.

2. Appareil selon la revendication 1, caractérisé en ce que la bande perforée (2) est une bande sans fin guidée autour de deux tambours de renvoi (3 et 4) dont l'un est commandé.

3. Appareil selon les revendications 1 et 2, caractérisé en ce que la pièce tubulaire (14) est disposée entre les deux tambours de renvoi (3 et 4), contre le brin inférieur (2a) de la bande (2), à distance du tambour (3) situé en aval, dans le sens de marche de la bande.

4. Appareil selon les revendications 1 à 3, caractérisé en ce que la tension de la bande (2) est réglable et que la surface externe (13) de la pièce tubulaire (14), coopérant avec la bande, fait légèrement saillie vers le bas par rapport à un plan défini par une tangente aux deux tambours (3, 4).

5. Appareil selon une des revendications 1 à 4, caractérisé en ce qu'il comprend, sous le brin inférieur (2a) de la bande, une courroie de transport (1) pour les masses tombant en forme de gouttes, courroie qui circule à la même vitesse (v) que la bande perforée (2) et parallèlement au brin inférieur (2a) de celle-ci.

6. Appareil selon la revendication 5, caractérisé en ce que plusieurs pièces tubulaires (14, 14a) sont disposées à l'intérieur de la bande perforée (2).

7. Appareil selon une des revendications 1 à 6, caractérisé en ce que la bande perforée (2) est une bande en matière synthétique, de préférence en chlorure de polyvinyle armé.

8. Appareil selon la revendication 1, caractérisé en ce que la surface externe (13) de la pièce tubulaire (14), qui est en contact avec la bande (2), est bombée des deux côtés de la fente (15).

9. Appareil selon la revendication 1, caractérisé en ce que la pièce tubulaire (14) présente un canal (16) de guidage de la masse à transformer en gouttes, des orifices (18) disposés transversalement à ce canal en une rangée, ainsi qu'une rainure (19) qui relie ces orifices transversaux entre eux, rainure qui s'ouvre vers l'extérieur et forme la fente de sortie (15) par son côté débouchant dans la surface externe (13).

10. Appareil selon la revendication 9, caractérisé en ce que des canaux (21) pour la circulation d'une huile de chauffage, ou d'autres éléments de chauffage pour le maintien à température de la masse sortante, sont disposés parallèlement au canal (16) d'amenée de la masse.

11. Appareil selon une des revendications 1 à 10, caractérisé en ce que la pièce tubulaire (14) est munie d'éléments de guidage (25) qui dirigent du produit sortant latéralement entre la pièce tubulaire (14) et la bande (2) vers les perforations.

12. Appareil selon une ou plusieurs des revendications 1 à 11, caractérisé en ce que la bande perforée (2) et la ou les pièces tubulaires (14, 14a) sont entourées d'un carter (22) dont la température intérieure est réglée.

13. Appareil selon une des revendications 1 à 12, caractérisé en ce que des appareils de chauffage par rayonnement (24) sont coordonnés au trin supérieur (2b) de la bande perforée (2) et au tambour de renvoi (4) situé à la fin de ce brin dans le sens de marche.

14. Appareil selon une des revendications 1 à 13, caractérisé en ce que les deux tambours de renvoi (3 et 4) sont parcourus par un fluide chauffant.

15. Appareil selon les revendications 1 et 2, caractérisé en ce qu'une paroi formant barrage (25, 25') s'étend transversalement à la bande sur la largeur de celle-ci et est appuyée par son bord inférieur sur le brin supérieur (2b) de la bande (2), afin de presser vers l'intérieur, à travers les perforations, le matériau qui adhère encore au côté externe de la bande (2).

16. Appareil selon la revendication 15, caractérisé en ce que la paroi (25) est constituée d'éléments (25a, 25b) formant un entonnoir qui s'ouvre en sens contraire au sens de marche (5b) du brin supérieur (2b) de la bande.

17. Appareil selon la revendication 16, caractérisé en ce que les éléments de paroi (25a, 25b) sont des faces planes inclinées sous un angle (α) l'une par rapport à l'autre.

18. Appareil selon la revendication 17, caractérisé en ce que l'angle (α) est un angle obtus.

19. Appareil selon une des revendications 16 à 18, caractérisé en ce que le sommet (26) de l'angle (α) est situé au milieu de la bande et que les deux éléments de paroi (25a, 25b) s'étendent symétriquement par rapport au milieu de la bande.

20. Appareil selon les revendications 15 et 16, caractérisé en ce que les éléments de paroi (25') sont conformés de manière que des tangentes, tracées sur ces éléments à proximité de la surface de la bande, forment avec cette surface un angle (β) aigu qui s'ouvre en sens contraire au sens de marche (5b) du brin supérieur (2b) de la bande.

21. Appareil selon la revendication 20, caractérisé en ce que les éléments de paroi (25') sont plans et sont inclinés par rapport à la surface de la bande.

22. Appareil selon une des revendications 15 à 21, caractérisé en ce que les éléments de paroi (25a, 25b, 25') sont disposés dans la région du tambour de renvoi (3) situé en amont dans le sens de marche (5b) du brin supérieur (2b) de la bande.

23. Appareil selon une des revendications 15 à 21, caractérisé en ce qu'un bord racleur (27a), s'étendant transversalement au sens de marche (5b), attaque le côté intérieur du brin supérieur (2b) dans la région du tambour de renvoi (4) situé en aval dans le sens de marche (5b) de ce brin de la bande.

24. Appareil selon la revendication 22, caractérisé en ce que le bord racleur (27a) est le bord supérieur d'une paroi d'égouttage (27) qui est située en amont de la pièce tubulaire (14), vu dans le sens de marche (5a) du brin inférieur (2a) de la bande.

25. Appareil selon les revendications 23 et 24, caractérisé en ce que la paroi d'égouttage (27) est munie, à son bord inférieur, d'une gouttière collectrice (28) pour le matériau s'écoulant le long de cette paroi.

0 134 944

**FIG.1**

**FIG.2**

**FIG.3**

**FIG.4**

0 134 944

# FIG. 5

# FIG. 6

# FIG. 7

3

0 134 944

FIG. 8

FIG. 9

FIG. 11

FIG. 10

5